# EUROPEAN PATENT APPLICATION

(11) **EP 1 733 738 A1**
(43) Date of publication of application: **20.12.2006**
(21) Application number: 05721547.7
(22) Date of filing: 18.03.2005
(51) Int. Cl.: A61K 45/00

(54) **METHOD OF PREVENTING ULTRAVIOLET RAY-INDUCED APOPTOSIS**

(30) Priority: 24.03.2004 JP 2004087051
(71) Applicant: SHISEIDO COMPANY, LTD., Tokyo 104-8010 (JP)
(72) Inventor: KATAGIRI, Chika c/o SHISEIDO RESEARCH CENTER, Yokohama-shi, Kanagawa 2368643 (JP); HIBINO, Toshihiko c/o SHISEIDO RESEARCH CENTER, Yokohama-shi, Kanagawa 2368643 (JP)
(74) Representative: Santarelli, Luc
(86) International application number: PCT/JP2005/005620
(87) International publication number: WO 2005/089801

(57) **Abstract**

The present invention provides a method for enhancing resistance to the onset of skin damage caused by irradiation of the skin with ultraviolet light by inhibiting ultraviolet irradiation-induced apoptosis of epidermal cells as a result of increasing expression of SCCA-1 and/or SCCA-2 in epidermal cells. Moreover, the present invention provides a skin testing method for evaluating resistance of the skin to skin damage caused by ultraviolet irradiation by measuring the expression of SCCA-1 and/or SCCA-2 in epidermal cells, and judging UV resistance of the epidermis to be weak in the case expression is decreased as compared with expression in epidermal cells. Moreover, the present invention provides a method for screening drugs which enhance resistance of the skin to skin damage by ultraviolet irradiation by selecting a drug that increases expression of SCCA-1 and/or SCCA-2 as a UV resistance enhancer.

SEQUENCE LISTING
<110> SHISEIDO CO. LTD.
<120> Method for Reducing Ultraviolet Light Induced Apoptosis
<130> 1034468
<140> JP 2004-087051
<141> 2004-03-24
<160> 13

<210> 1
<211> 17
<212> DNA
<213> Artificial Sequence
<223> Forward Primer

<400> 1
gtgctatctg gagtcct 17

<210> 2
<211> 16
<212> DNA
<213> Artificial Sequence
<223> Reverse Primer

<400> 2
ctgttgttgc cagcaa 16

<210> 3
<211> 17
<212> DNA
<213> Artificial Sequence
<223> Taq Man Probe

<400> 3
catcacctac ttcaact 17

<210> 4

<211> 24
<212> DNA
<213> Artificial Sequence
<223> Forward Primer

<400> 4
ctctgcttcc tctaggaac acag                                                24

<210> 5
<211> 20
<212> DNA
<213> Artificial Sequence
<223> Reverse Primer

<400> 5
tgttggcgat cttcagctca                                                    20

<210> 6
<211> 21
<212> DNA
<213> Artificial Sequence
<223> Taq Man Probe

<400> 6
agttccagat cacatcgagt t                                                  21

<210> 7
<211> 19
<212> DNA
<213> Artificial Sequence
<223> Forward Primer

<400> 7
gaaggtgaag gtcggagtc                                                     19

<210> 8

<211> 20
<212> DNA
<213> Artificial Sequence
<223> Reverse Primer

<400> 8
gaagatggtg atgggatttc                                                    20

<210> 9
<211> 22
<212> DNA
<213> Artificial Sequence
<223> Taq Man Probe

<400> 9
aggctgagaa cgggaagctt gt                                                 22

<210> 10
<211> 21
<212> DNA
<213> Artificial Sequence
<223> SiRNA Target Sequence

<400> 10
acatgaactt ggtgttggct t                                                  21

<210> 11
<211> 65
<212> DNA
<213> Artificial Sequence
<223> Sense Oligonucleotide for SiRNA

<400> 11
gatcccggcc aacaccaagt tcatgtttca agagaacatg aacttggtgt tggctttttt        60
ggaaa                                                                    65

<210> 12
<211> 21
<212> DNA
<213> Artificial Sequence
<223> SiRNA Target Sequence

<400> 12
aagccaacac caagttcatg t                                                21

<210> 13
<211> 65
<212> DNA
<213> Artificial Sequence
<223> Antisense Oligonucleotide for SiRNA

<400> 13
agcttttcca aaaaagccaa caccaagttc atgttctctt gaaacatgaa cttggtgttg      60
gccgg                                                                  65

## Description

### TECHNICAL FIELD

The present invention relates to a means for enhancing the ultraviolet (UV) defense mechanism of skin by increasing expression of squamous cell carcinoma antigens (SCCA-1 and SCCA-2).

### BACKGROUND ART

The epidermis is known to be subjected to various damage by ultraviolet (UV) irradiation. Although pigmentation occurs when the skin is irradiated with ultraviolet light, if repeatedly subjected to continuous irradiation, the epidermis hypertrophies and loses elasticity. Known symptoms of damage to the epidermis caused by ultraviolet light include inflammation, rough skin, wrinkles, sagging, pigmentation, liver spots, darkening, moles, purpura and capillary dilation, and these conditions may lead to malignant tumors. Ultraviolet skin damage typically occurs frequently at areas of the skin routinely exposed to sunlight, such as the face, neck and limbs. Skin damage caused by ultraviolet irradiation is viewed as a problem due to destruction of the ozone layer in particular.

Findings relating to the physiological UV defense mechanism of the skin have come about primarily from research on melanin produced by melanocytes of the basal layer, and searches for and development of antioxidants. In addition, radiation and ultraviolet radiation is known to induce apoptosis (Masayuki Miura, Takeshi Yamada, ed., Experimental Medicine Supplement, "Term Library - Apoptosis", 1996), and research has also been conducted on anti-apoptosis protein bc1-2 in basal cells (Fang, et al., J. Immunol. 1994, 153, 4388-4398; Takahashi, et al., Photochem. Photobiol. 2001, 74(4), 579-586). Protein bc1-2 is believed to demonstrate a UV defensive function by blocking or delaying apoptosis. However, there have been very few findings obtained relating to intrinsic UV defense proteins in the upper layer of the epidermis.

### DISCLOSURE OF THE INVENTION

An object of the present invention is to provide a novel and effective UV defense means by elucidating a new epidermis-specific UV defense mechanism.

Psoriasis is a skin disease in the form of a chronic, recurrent inflammatory parakeratosis characterized by proliferation and differentiation abnormalities of epidermal cells and invasion by inflammatory cells (Hopso-Havu et al., British Journal of Dermatology (1983), 109, 77-85). Psoriasis is believed to occur due to the involvement of genetic factors in combination with various environmental factors. It is known that when epidermis in which psoriasis has occurred is compared with normal epidermis, increased expression of squamous cell carcinoma antigen (SCCA) is observed in the upper layer of the psoriatic epidermis (Takeda A. et al., J. Invest. Dermatol. (2002), 118(1), 147-154). SCCA is an antigen which was discovered in squamous cell carcinoma cells of the cervix, exhibits high concentrations in the blood in squamous cell carcinoma of the cervix, lungs, esophagus and skin, and is frequently used to diagnose squamous cell carcinoma and assess therapeutic effects. However, in research conducted by the inventor of the present invention, expression of SCCA in squamous cell carcinoma of the skin was observed to be not so high. SCCA is encoded by two genes, SCCA-1 and SCCA-2, arranged in tandem, on chromosome 18q21.3. Both SCCA-1 and SCCA-2 are proteins having a molecular weight of about 45,000, and although have an extremely high degree of homology, have different amino acid sequences at the active site, and are therefore believed to have different functions (Schick et al., J. Biol. Chem. (1997), 27213, 1849-55).

The inventor of the present invention conducted a study of the skin's UV defense mechanism by focusing on SCCA-1 and SCCA-2. As a result of employing immunohistological methods and in situ hybridization, the expression of SCCA at exposed sites of skin was determined to increase remarkably as compared with skin at non-exposed sites. Moreover, expression of SCCA was determined to be remarkably increased in the prickle cell layer and granular layer as a result of irradiating human skin with UV light. The protein expression was not observed in the basal layer. In addition, increased expression of SCCA induced by UV irradiation was similarly observed in a three-dimensional skin model and cultured human keratinocytes.

The inventor of the present invention next established stable expression systems by introducing human SCCA-1 and SCCA-2 genes into 3T3 cells in which expression of SCCA is not observed. As a result of analyzing by FACS (fluorescence activated cell sorting) using Annexin V-FITC and propidium iodine (PI) as indicators of apoptosis, apoptosis caused by UV irradiation was determined to decrease significantly in both of the SCCA stable expression systems.

Moreover, SCCA-1 and SCCA-2 knocked down cell lines (siSCCA) were established by RNA interference using a pSilencer vector, by which HaCat cells which highly express SCCA are rendered to constantly express SiRNA. SCCA expression in the siSCCA cell lines was confirmed by quantitative PCR to be inhibited by 90% or more. As a result of irradiating with UV light at 75 mJ/cm², in contrast to apoptosis occurring in 38% of the cells of a control line, apoptosis was determined to be induced in about 80% of the cells in the siSCCA cell lines.

In summary of the above results, SCCA was clearly determined to be a protein that plays an important role in UV defense mechanism.

The present invention was established on the basis of the aforementioned findings, and in a first aspect thereof, provides a method for inhibiting apoptosis of epidermal cells by increasing expression of SCCA-1 and/or SCCA-2 in epidermal cells. In a preferable aspect thereof, the apoptosis is apoptosis induced by ultraviolet irradiation. Thus, the method of the present invention is able to enhance resistance to the onset of skin damage caused by ultraviolet irradiation of the skin. The epidermal cells include keratinocytes, granular cells, prickle cells and the like.

In another aspect, the present invention provides a method for testing skin by evaluating resistance to skin damage caused by ultraviolet irradiation of the skin (to be referred to as "UV resistance"). This method is characterized by assessing UV resistance of the skin to be weak in the case expression of SCCA-1 and/or SCCA-2 in epidermal cells has decreased in comparison with expression of epidermal cells.

Preferably, increased expression of SCCA-1 and/or SCCA-2 in epidermal cells is determined by measuring the amounts of SCCA-1 and/or SCCA-2 in the epidermal cells. This measurement can be carried out by, for example, ELISA or RIA using antibody specific to SCCA-1 and/or SCCA-2. In addition, increased expression of SCCA-1 and/or SCCA-2 in epidermal cells may also be determined by measuring the amount of mRNA encoding SCCA-1 and/or SCCA-2 in the epidermal cells. This measurement of mRNA can be carried out by polymerase chain reaction (PCR), and preferably by real-time PCR.

In still another aspect of the present invention, the present invention provides a method for screening drugs which enhance resistance to skin damage caused by ultraviolet irradiation of skin (to be referred to as "UV resistance enhancers"). This method is characterized by selecting a drug which increases expression of SCCA-1 and/or SCCA-2 for use as a UV resistance enhancer.

Thus, according to the present invention, it is possible to provide a method for enhancing UV resistance of skin, a method for testing UV resistance of skin, and a novel UV resistance enhancer.

### BRIEF DESCRIPTION OF THE DRAWINGS

FIG. 1 shows the expression of SCCA in skin at an exposed site and skin at a non-exposed site;
FIG. 2 shows fluctuations in the expression of SCCA in skin caused by UV irradiation;
FIG. 3 shows the effects of UV irradiation on SCCA expression in cultured human keratinocytes;
FIG. 4 shows a comparison of the rates of apoptosis induced by UV irradiation between SCCA highly expressing cells and SCCA non-expressing cells;
FIG. 5 shows the establishment of an SCCA knocked down cell line; and
FIG. 6 shows a comparison of the rates of apoptosis induced by UV irradiation between SCCA knocked down cells and control cells.

### BEST MODE FOR CARRYING OUT THE INVENTION

As previously described, since there have been no reports suggesting the presence of a relationship between SCCA and ultraviolet irradiation, the inhibition of ultraviolet-induced apoptosis by SCCA is a surprising fact first discovered by the inventor of the present invention.

The present invention, in a first aspect thereof, provides a method for inhibiting apoptosis of epidermal cells, and preferably apoptosis induced by ultraviolet irradiation, by increasing the expression of SCCA-1 and/or SCCA-2 of epidermal cells. This method makes it possible to enhance resistance to the onset of skin damage caused by ultraviolet irradiation of the skin.

As previously described, SCCA is a protein having a molecular weight of about 45,000 which is present in squamous cell carcinoma cells of the uterus and the like and in psoriatic skin. The amino acid sequences of SCCA-1 and SCCA-2 along with the amino acid sequences which encode them are described in Takeda, A. et al., J. Invest. Dermatol. 118, 147-154 (2002) (op. cit.). Increased expression of SCCA-1 and/or SCCA-2 in epidermal cells can be achieved by, for example, applying a reagent which increases expression thereof. SCCA-1 and/or SCCA-2 itself may be used for said reagent.

When the gene which encodes SCCA-1 and/or SCCA-2 in a specimen is in an inactive state or silent state, and as a result, when cells are in a state in which SCCA-1 and/or SCCA-2 is deficient, increased expression of SCCA-1 and/or SCCA-2 can be achieved by introducing SCCA-1 and/or SCCA-2 gene itself into epidermal cells, or by placing a regulatory sequence which increases expression of SCCA-1 and/or SCCA-2 gene, such as a promoter or enhancer, at a location operably linked to those genes.

Examples of methods which can be applied to introduce a gene encoding SCCA-1 and/or SCCA-2 into cells include gene introduction using a virus vector and non-viral gene insertion (Nikkei Science, April 1994 edition, pages 20-45; Experimental Medicine Special Issue, 12(15) (1994); Experimental Medicine Supplement, "Basic Technologies of Gene Therapy", Yodosha Co., Ltd. (1996)). Examples of gene introduction methods using a virus vector include methods using DNA viruses such as retrovirus, adenovirus, adeno-associated virus, herpes virus, vaccinia virus, pox virus, polio virus or simbis virus, or methods in which DNA encoding SCCA-1 and/or SCCA-2 is incorporated into an RNA virus. Methods using a retrovirus, adenovirus, adeno-associated virus or vaccinia virus are particularly preferable. Examples of non-viral gene introduction methods include methods in which an expression plasmid is administered directly into muscle (DNA vaccine method), liposome method, lipofection, microinjection, calcium phosphate method and electroporation, with the DNA vaccine method and liposome method being particularly preferable. In addition, in vivo methods, in which DNA is introduced directly into the body, and ex vivo methods, in which a certain type of cells are removed from a human body, DNA is inserted into the cells outside the body, and then the cells are returned to the body, (Nikkei Science, April 1994 edition, pages 20 to 45; Pharmaceuticals Monthly, 36(1), 23-48 (1994); Experimental Medicine Special Issue, 12(15) (1994)) can be used to allow the aforementioned gene to actually act as a pharmaceutical, with in vivo methods being more preferable. In the case of administering using an in vivo method, administration can be carried out using a suitable administration route corresponding to the disease, symptoms and so forth. Examples of administration routes include intravenous, intraarterial, subcutaneous, intracutaneous and intramuscular administration. In the case of administering by an in vivo method, the gene is typically used in the form of an injection preparation, and a commonly used carrier may be added as necessary. In addition, in the case of administering in the form of a liposome or fusogenic liposome (such as Sendai virus (HVJ)-liposome), the gene can be used in the form of a liposome preparation such as a suspension, frozen preparation or centrifugally concentrated frozen preparation.

In a second aspect of the present invention, the present invention provides a method for testing skin by evaluating UV resistance of skin. This method is characterized by assessing UV resistance of the skin to be weak in the case expression of SCCA-1 and/or SCCA-2 in epidermal cells has decreased in comparison with expression of epidermal cells.

For example, evaluation criteria may consist of judging UV resistance of skin to be weak if expression of SCCA-1 and/or SCCA-2 in epidermal cells has decreased by 10% or more, 20% or more, 30% or more, 50% or more, 70% or more or by 100% as compared with a control value. The control value may be, for example, the average value of the expressed amount of SCCA-1 and/or SCCA-2 of epidermal cells at a corresponding site in a statistically significant number of healthy persons (e.g., 10 or more persons, and preferably 100 or more persons).

Increased SCCA-1 and/or SCCA-2 in epidermal cells can be determined by, for example, directly measuring the amount of SCCA-1 and/or SCCA-2 in epidermal cells. This measurement can be preferably carried out by a commonly known method in the industry which uses antibody specific to SCCA-1 and/or SCCA-2, examples of which include immunostaining methods using a fluorescent substance, pigment or enzyme, western blotting, immunoassay methods such as ELISA or RIA, and various other methods. In addition, the amount of SCCA-1 and/or SCCA-2 can also be determined by extracting mRNA from the skin and measuring the amount of mRNA which encodes SCCA-1 and/or SCCA-2. Extraction of mRNA and measurement of the amount thereof is commonly known in the industry, and for example, quantification of RNA is carried out by quantitative polymerase chain reaction (PCR). In addition, the expressed amount of SCCA-1 and/or SCCA-2 can also be measured by measuring known biological activity of SCCA-1 and/or SCCA-2. Moreover, expression of SCCA-1 and/or SCCA-2 can be determined by in situ hybridization or through measurement of the biological activity thereof.

Moreover, the present invention provides a method for screening UV resistance enhancers which enhance resistance to skin damage caused by ultraviolet irradiation of skin. This method is characterized by selecting a drug which increases expression of SCCA-1 and/or SCCA-2 for use as a UV resistance enhancer. As previously described, increased expression of SCCA-1 and/or SCCA-2 can be determined by measuring the amount of SCCA-1 and/or SCCA-2 in cells, or measuring the amount of mRNA which encodes SCCA-1 and/or SCCA-2 extracted from epidermal cells.

In a preferable aspect of the present invention, the aforementioned screening method comprises the application of a candidate drug having the aforementioned increasing ability to a model animal, and preferably an animal deficient in SCCA-1 and/or SCCA-2, and selecting a candidate drug which enhances UV resistance.

Evaluation of UV resistance of epidermal cells can be carried out by, for example, assessing activity which inhibits apoptosis induced by irradiating epidermal cells with ultraviolet light. Evaluation of apoptosis can be carried out according to methods commonly known among persons skill in the art. For example, apoptosis can be evaluated by using the FACS Coulter Counter (EPIX XL-MCL, Beckman Coulter) and carrying out an FACS analysis using the Annexin V-FITC and propidium iodine (PI) double staining method (Annexin V-FITC Kit, Immunotech) as an indicator. Evaluation criteria may be such that UV resistance of skin is judged to be enhanced if, for example, apoptosis of epidermal cells is inhibited by 30% or more, preferably 50% or more, more preferably 70% or more, and most preferably 90% or more.

The following provides a more detailed explanation of the present invention through specific examples thereof. Furthermore, the present invention is not limited to these examples.

### Immunohistochemical Examination

Biopsied epidermis was fixed in cold acetone and then embedded in paraffin in accordance with the AMeX procedure (Sato Y. et al., Am. J. Pathol., 125, 431-435 (1986)). Sections were deparaffined, washed with acetone and then PBS. Next, non-specific binding sites of the sections were blocked with 10% normal goat serum (Histofine, Tokyo, Japan).

The epidermal sections were then respectively incubated in anti-SCCA-1 monoclonal antibody (Santa Cruz Biotechnology, CA, USA; 1:500 dilution), anti-SCCA-2 monoclonal antibody (Santa Cruz Biotechnology, CA, USA; 1:500 dilution) or anti-SCCA polyclonal antibody (purified in the manner described in Takeda A. et al., J. Invest. Dermatol., 118, 147-154 (2002)). After washing with PBS, the sections were counter-stained with hematoxylin and observed with the DAKO Envision System (Dako Corp., CA, USA).

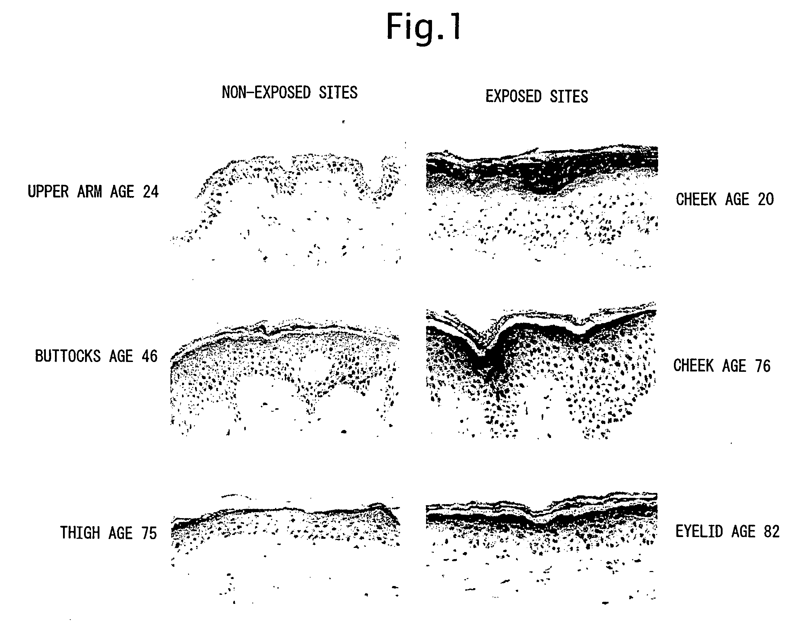

Fig. 1 shows the results of sampling epidermis from non-exposed sites consisting of the upper arm (human, 24 years old), buttocks (human, 46 years old) and thigh (human: 75 years old), and exposed sites consisting of the cheek (human, 20 years old, 76 years old) and eyelid (human, 82 years old), followed by microscopic observations using anti-SCCA polyclonal for the antibody, which binds to both SCCA-1 and SCCA-2. It can be seen from Fig. 1 that SCCA had increased considerably in the epidermal upper layer of the exposed sites as compared with the non-exposed sites. However, expression of SCCA was not observed to increase in the basal layer of the exposed sites.

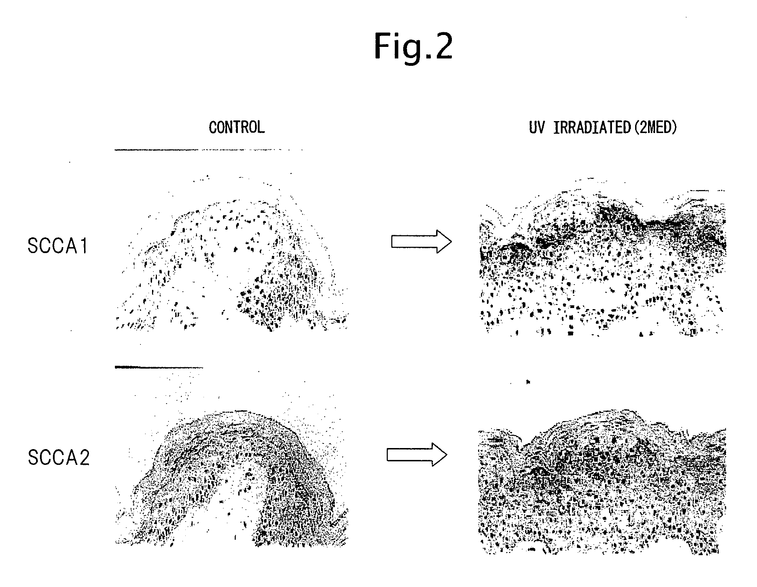

Fig. 2 shows the results of microscopic observation of the respective expression of SCCA-1 and SCCA-2 in epidermis specimens consisting of human epidermis subjected to UV irradiation (Transluminator TOREX FL205-E-30/DMR (Toshiba Medical Supply) at a dose of 2 MED (Minimum Erythema Dose), and control epidermis not subjected to UV irradiation. Anti-SCCA-1 monoclonal antibody and anti-SCCA-2 monoclonal antibody were respectively used for the antibodies. As can be seen from Fig. 2, expression of both SCCA-1 and SCCA-2 clearly increased due to UV irradiation of human epidermis. In addition, the increase in expression was prominent in the epidermal prickle cell layer and granular layer.

On the basis of the above, expression of SCCA-1 and SCCA-2 in the epidermis, and particularly in the prickle cell layer and granular layer thereof, were clearly determined to increase following UV irradiation of the epidermis.

### Quantitative PCR Experiment

Next, an experiment was conducted to confirm that expression of SCCA-1 and SCCA-2 in the epidermis is increased by UV irradiation.

Human keratinocytes were cultured in keratinocyte-SFM medium (Gibco, Invitrogen Corp.) in the presence of L-glutamine and epithelial cell growth factor at high humidity, temperature of 37°C and in a 5% CO₂ atmosphere. Cells that had reached a cell density of 60 to 70% were irradiated with UVB for 0 to 48 hours. UVB irradiation was carried out using the Transluminator TOREX FL205-E-30/DM (Toshiba Medical Supply) at an intensity of 30 mJ/cm². Control cells were not irradiated with UVB.

Total RNA from the aforementioned cultured cells was isolated and purified using Isogen (Nippon Gene) according to the instructions provided therein. The expression levels of SCCA-1 and SCCA-2 were respectively determined by quantitative real-time polymerase chain reaction (PCR). In brief, the total RNA was transcribed to cDNA using Superscript II (Invitrogen, Carlsbad, CA). The samples were then amplified by carrying out a 2-step PCR with 40 cycles using the ABI PRISM 7900HT Sequence Detection System (Applied Biosystems, Foster City, CA). G3PDH (glycerylaldehyde-3-phosphate dehydrogenase) was used as the internal standard.

The primers used are indicated below.
SCCA-1:
   Forward primer:
      5'-GTGCTATCTGGAGTCCT-3' (SEQ ID NO. 1)
   Reverse primer:
      5'-CTGTTGTTGCCAGCAA-3' (SEQ ID NO. 2)
   Taq Man probe:
      5'-CATCACCTACTTCAACT-3' (SEQ ID NO. 3)
SCCA-2:
   Forward primer:
      5'-CTCTGCTTCCTCTAGGAACACAG-3' (SEQ ID NO. 4)
   Reverse primer:
      5'-TGTTGGCGATCTTCAGCTCA-3' (SEQ ID NO. 5)
   Taq Man probe:
      5'-AGTTCCAGATCACATCGAGTT-3' (SEQ ID NO. 6)
G3PDH:
   Forward primer:
      5'-GAAGGTGAAGGTCGGAGTC-3' (SEQ ID NO. 7)
   Reverse primer:
      5'-GAAGATGGTGATGGGATTTC-3' (SEQ ID NO. 8)
   Taq Man probe:
      5'-AGGCTGAGAACGGGAAGCTTGT-3' (SEQ ID NO. 9)

A reporter dye (6-carboxyfluoroscein) was coupled to the 5' end of the Taq Man probe sequence, and a quencher dye (6-carboxytetramethyl rhodamine) was incorporated on the 3' end.

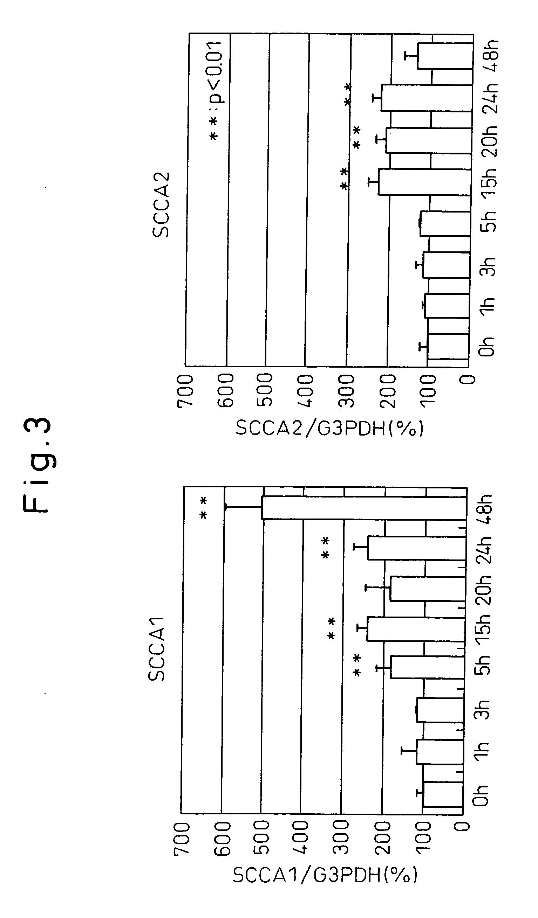

Fig. 3 shows the results of the effects of UVB irradiation on expression of SCCA in cultured human keratinocytes. Expression of both SCCA-1 and SCCA-2 was clearly determined to increase as a result of UV irradiation. Thus, expression of SCCA-1 and SCCA-2 was clearly demonstrated to increase at the gene level due to UV irradiation of epidermal cells.

### Study of Role of SCCA in UV Irradiation

On the basis of the above, expression of SCCA-1 and SCCA-2 in epidermal cells was determined to increase when subjected to UV irradiation. Next, a study was conducted as to the role played by SCCA-1 and SCCA-2 in epidermal cells subjected to UV irradiation.

### Establishment of SCCA-1 and SCCA-2 Highly Expressing Cells

3T3 cells (acquired from ATCC) are cells originating in the fetus of mice which do not express human SCCA-1 or SCCA-2. Genes encoding SCCA-1 or SCCA-2 were introduced into these cells in the manner described below.

SCCA-1 and SCCA-2 cDNA (Takeda, et al., J. Invest. Dermatol., 118, 147-154 (2002)) was digested twice with BamHI and KpnI. These digestion products were subcloned in a pTarget vector and transfected into 3T3 cells using Lipofectamine Plus (Gibco, Invitrogen Corp.). In brief, 20 µg of cDNA were mixed with 75 µl of Plus reagent in 675 µl of serum-free DMEM medium (Invitrogen Corp.) and allowed to stand for 15 minutes at 25°C. Lipofectamine (100 µl) was then added to 650 µl of the serum-free DMEM medium followed by addition of the aforementioned cDNA-Plus mixture and allowing to stand for 15 minutes at 25°C. This cDNA mixture was added to 10 ml of serum-free DMEM medium followed by incubation of the 3T3 cells therein for 4 hours at 37°C in a 5% CO₂ atmosphere. The medium was exchanged to a DMEM medium containing 10% FCS (Invitrogen Corp.), and then incubated overnight. On the following day, G418 (Calbiochem) was added to a final concentration of 500 µg/ml. The concentration of the G418 was maintained during the culturing period. The medium was replaced every 2 to 3 days. After culturing for 4 weeks, several of the G418-resistant colonies were able to be isolated, enabling the establishment of SCCA-1- and SCCA-2-expressing cell lines.

Cells transfected with cDNA encoding SCCA-1 (SCCA-1 inserted cells) were confirmed to specifically and stably express SCCA-1, while cells transfected with cDNA encoding SCCA-2 (SCCA-2 inserted cells) were confirmed to specifically and stably express SCCA-2. In addition, 3T3 cells transfected with a non-specific sequence using the same procedure (control cells) did not express SCCA-1 or SCCA-2.

The roles of SCCA-1 and SCCA-2 when subjected to UV irradiation were studied using the aforementioned SCCA-1 inserted cells, SCCA-2 inserted cells and control cells. More specifically, a study was conducted of the roles of SCCA-1 and SCCA-2 against UV-induced apoptosis in epidermal cells.

Each of the aforementioned cells were cultured in DMEM medium containing 10% FCS at high humidity, temperature of 37°C and in a 5% CO₂ atmosphere. Those cells that had reached a cell density of 60 to 70% were irradiated with UVB for 0 to 48 hours. UVB irradiation was carried out using the Transluminator TOREX FL205-E-30/DMR (Toshiba Medical Supply) at an intensity of 30 mJ/cm².

Evaluation of apoptosis in these cells was carried out by FACS (fluorescence activated cell sorting) analysis with the FACS Coulter Counter (EPIX XL-MCL, Beckman Coulter) using Annexin V-FITC and propidium iodine (PI) double staining (Annexin V-FITC Kit, Immunotech) as the indicator of apoptosis.

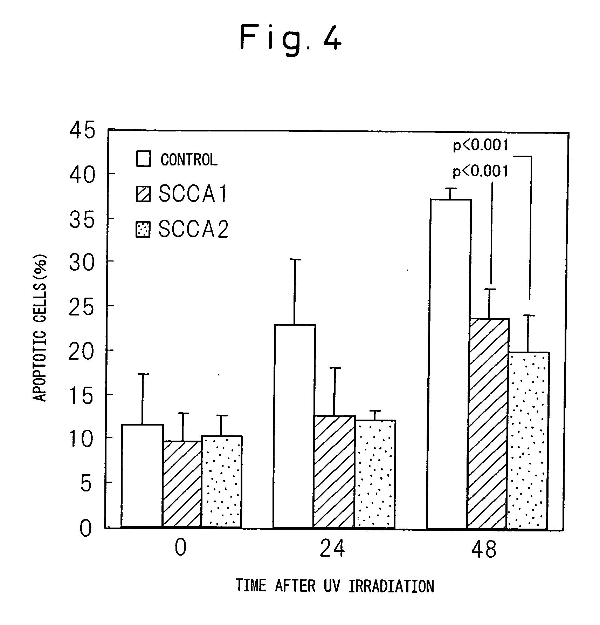

Those results are shown in Fig. 4. As is clear from Fig. 4, apoptosis induced by UV irradiation was observed to decrease significantly in both cells transfected with SCCA-1 and cells transfected with SCCA-2. Thus, both SCCA-1 and SCCA-2 are presumed to be able to inhibit UV-induced apoptosis.

In order to confirm this finding, the inventor of the present invention next established SCCA-1 and SCCA-2 knocked down cell lines by RNA interference to assess the roles of SCCA-1 and SCCA-2 in epidermal cells during UV irradiation.

### Establishment of SCCA Knockdown Cells

HaCat cells (H. Hans, et al., Experimental Cell Research 239, 399-410 (1998)) are human keratinocytes which highly express SCCA. SCCA-1 and SCCA-2 knocked down cell lines were established by rendering them to constantly express siRNA (short interference RNA) by means of a pSilencer vector (Ambion) in accordance with RNA interference.

The siRNA was constructed using the pSilencer vector according to the instructions provided. More specifically, a double-strand oligonucleotide, comprised of a 65 mer sense oligonucleotide (SEQ ID NO. 11) containing a 21 mer oligonucleotide complementary to the locations of nucleotide nos. 46 to 66 of a gene encoding SCCA (ACATGAACTT GGTGTTGGCT T: SEQ ID NO. 10), and a 65 mer antisense oligonucleotide (SEQ ID NO. 13) containing a 21 mer oligonucleotide homologous to the locations of nucleotide nos. 46 to 66 (AAGCCAACAC CAAGTTCATG T: SEQ ID NO. 12), was cloned to the Hind III site and Bam HI site of the pSilencer vector. Transfection into HaCat cells was carried out using Lipofectamine 2000 (Invitrogen) according to the instructions provided therein. Control cells were prepared using a double-strand oligonucleotide not having significant homology or complementarity with mammalian gene sequences. Stable cells lines were acquired by culturing the transfected cells for 4 to 6 weeks in hygromycin B medium and selecting the cells. Confirmation of inhibition of SCCA expression was carried out in the aforementioned manner, and expression of SCCA-1 and SCCA-2 was measured by real-time PCR.
Sense oligonucleotide (SEQ ID NO. 11):
   GATCCCGGCCAACACCAAGTTCATGTTTCAAGAGA
   ACATGAACTTGGTGTTGGCTTTTTTGGAAA (underline indicates homologous region)
Antisense oligonucleotide (SEQ ID NO. 13):
   AGCTTTTCCAAAA AAGCCAACACCAAGTTCATGT
   TCTCTTGAAACATGAACTT GGTGTTGGCCGG (underline indicates complementary region)

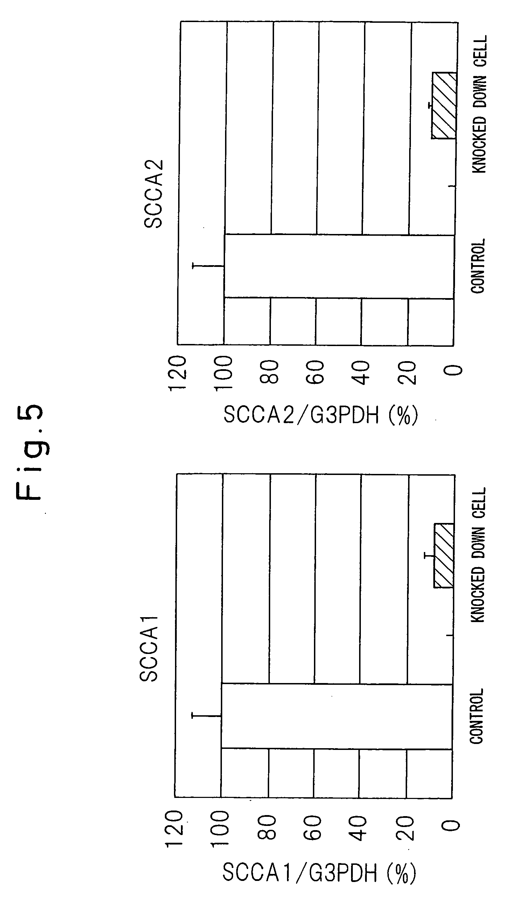

Those results are shown in Fig. 5. In the cells transfected with the aforementioned siRNA, expression of SCCA-1 and SCCA-2 was confirmed to be inhibited (knocked down) by 90% or more as compared with the control cells.

A study was then conducted on the roles of SCCA-1 and SCCA-2 against UV-induced apoptosis in epidermal cells using the aforementioned knockdown cells and control cells.

Each of the cells were incubated at high humidity and at a temperature of 37°C in a 5% CO₂ atmosphere in keratinocyte SFM medium (Gibco, Invitrogen) in the presence of L-glutamine and epithelial cell growth factor. Those cells that had reached a cell density of 60 to 70% were irradiated with UVB. UVB irradiation was carried out using the Transluminator TOREX FL205-E-30/DMR (Toshiba Medical Supply) at an intensity of 75 mJ/cm².

Evaluation of apoptosis in these cells was carried out by FACS (fluorescence activated cell sorting) analysis with the FACS Coulter Counter using Annexin V-FIT and propidium iodine (PI) double staining as the indicator of apoptosis.

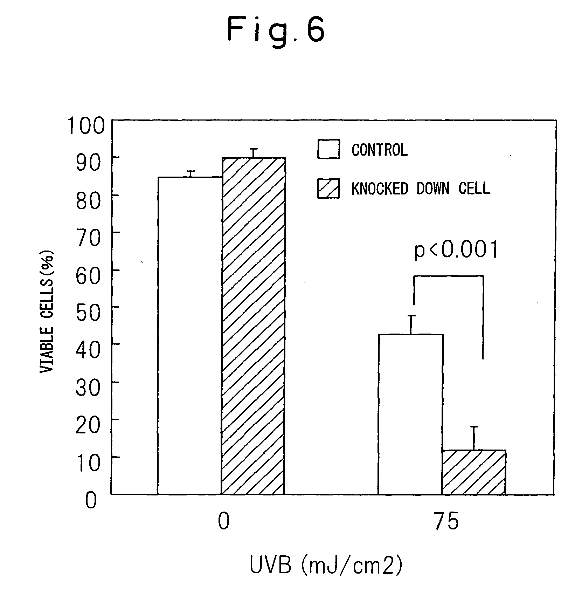

Those results are shown in Fig. 6. As a result of irradiating the knocked down cells with UV light, in contrast to apoptosis occurring in 38% of the control cells, apoptosis was clearly demonstrated to be induced in about 80% of the knocked down cells. Thus, SCCA was determined to significantly inhibit apoptosis of epidermal cells induced by UV irradiation. Accordingly, SCCA is believed to be responsible for the UV defense mechanism of epidermal cells.

## Claims

1. A method for inhibiting apoptosis of epidermal cells by increasing expression of SCCA-1 and/or SCCA-2 in epidermal cells.

2. The method according to claim 1, wherein the apoptosis is induced by ultraviolet irradiation, and resistance to the onset of skin damage by UV irradiation of the skin is enhanced by said method.

3. A method for testing skin by evaluating resistance to skin damage caused by ultraviolet irradiation of the skin, comprising: measuring the amounts of SCCA-1 and/or SCCA-2 in epidermal cells, and judging ultraviolet resistance of the skin to be weak in the case said amounts are decreased as compared with expression in epidermal cells.

4. The testing method according to claim 3, wherein expression of SCCA-1 and/or SCCA-2 is measured by ELISA or RIA using antibody specific to SCCA-1 and SCCA-2, respectively.

5. The testing method according to claim 3, wherein expression of SCCA-1 and/or SCCA-2 is measured by measuring the amount of mRNA encoding SCCA-1 and/or SCCA-2 extracted from epidermal cells.

6. A method for screening drugs which enhance resistance to skin damage caused by ultraviolet irradiation of skin, comprising: selecting a drug which increases expression of SCCA-1 and/or SCCA-2 for use as a ultraviolet resistance enhancer.
